Europäisches Patentamt

European Patent Office

Office européen des brevets

(11) Veröffentlichungsnummer: **0 145 863**
**A2**

## (12) EUROPÄISCHE PATENTANMELDUNG

(21) Anmeldenummer: **84111318.6**

(22) Anmeldetag: **22.09.84**

(51) Int. Cl.⁴: **B 01 L 7/00**, G 01 N 33/10,
F 27 B 17/02

(30) Priorität: **29.09.83 CH 5271/83**

(43) Veröffentlichungstag der Anmeldung: **26.06.85**
**Patentblatt 85/26**

(84) Benannte Vertragsstaaten: **CH DE FR GB IT LI**

(71) Anmelder: **Gebrüder Bühler AG, CH-9240 Uzwil (CH)**

(72) Erfinder: **Gavin, Michel, Dr., Hofbergstrasse 19,
CH-9500 Wil (CH)**
Erfinder: **Graenicher, Peter, Ruhbergstrasse 11,
CH-9230 Flawil (CH)**

(54) **Probenbehälter für Veraschungsproben.**

(57) Zur Schnellveraschung, insbesondere von Mehl, werden Probebehälter (1) verwendet, die vorzugsweise röhrchenartig ausgebildet sind. Für diese Probebehälter (1) ist eine stabförmige Dosiereinrichtung (8) vorteilhaft, und es ist ein Ofen (4) vorgesehen, der in seiner Ofenkammer (5) einen Träger (2) für mindestens einen Probebehälter (1) aufweist. Nach dem Schließen der Ofenkammer (5) wird ein Sauerstoff tragendes Gas durch die Ofenkammer (5) und die Probebehälter (1) von einer Zufuhröffnung (31) zu einer Abfuhröffnung (37) mit einer Durchsatzgeschwindigkeit von weniger als 10 l/min geblasen, wobei die Ofentemperatur oberhalb 750 °C und zweckmäßig bei 900 °C liegt.

## 010 PROBENBEHAELTER

Die Erfindung bezieht sich auf einen Probenbehälter für Veraschungsproben, vorzugsweise von, z.B. zerkleinertem, pflanzlichem Material, insbesondere von Mehl, bestehend aus einem gegen Temperaturen oberhalb 750°C hitzebeständigen Material, und einer in Seitenansicht mehrfach breiteren als hohen Form. Ferner bezieht sich die Erfindung auf einen Veraschungsofen für einen derartigen Probenbehälter bzw. einen Träger hierfür, auf eine Dosiereinrichtung für einen solchen Probenbehälter und auf ein Verfahren zur Schnellveraschung.

Die heute normierte Methode zur Veraschung geht von einer Veraschungstemperatur von 580 bis 900°C aus, wobei zur Verhinderung des Entstehens einer allzu starken Gasentwicklung bzw. Flammenbildung der in einem schüsselförmigen Probenbehälter, häufig aus Quarz, nur ein Minimum an Verbrennungssauerstoff zugeführt wird, weil sonst die Verbrennungsgase zu viel Asche mit sich reissen würden und das Ergebnis verfälschen könnten. Aus diesem Grunde benötigt man für die Veraschung mehrere Stunden, bevor das Ergebnis erhalten werden kann. Neben diesem Nachteil ergibt sich auch eine gewisse Ungenauigkeit dadurch, dass beim Oeffnen und Schliessen des Ofens zwangsläufig eine unkontrollierte Menge an Sauerstoff in die Ofenkammer gelangt und dort bis zum Verbrauch dieses Sauerstoffes eine stärkere Verbrennungsgasentwicklung verursacht.

Vielfach wird daher neben dieser Standardmethode zur Erzielung einer höheren Genauigkeit auch noch eine Verbrennung

- 2 -

mit niedrigerer Temperatur von z.B. 600°C durchgeführt. Es versteht sich, dass auch hier die Sauerstoffzufuhr nur langsam erfolgen darf, weshalb der Zeitaufwand bei niedrigerer Temperatur natürlich noch grösser ist.

In der DE-PS 929 450 wurde zwar bereits ein Schnellaschebestimmer vorgeschlagen, wobei die Erhitzung mit Hilfe einer Hochfrequenz-Heizeinrichtung erfolgen sollte. Diese Methode hat sich jedoch nicht durchgesetzt, weil dabei keineswegs eine Sicherung gegen allzu starke Verbrennungsgasentwicklung getroffen war, so dass die Genauigkeit bescheiden bleiben musste. Zudem musste der Sendeschwingkreis laufend nachgeregelt werden, um sich dem sich ändernden Widerstand der Probe während der Verbrennung anzupassen. Die Messung des Widerstandes konnte jedoch unter den gegebenen Bedingungen nur sehr ungenau erfolgen, was auch eine grosse Schwankung der Verbrennungstemperatur und somit eine weitere Unsicherheit mit sich brachte.

Zwar ist für die Verbrennung von tierischen Proben bereits ein Niedertemperaturofen vorgeschlagen worden, bei dem die Probe in einer Kapsel eingebracht und während der Verbrennung eine dosierte Menge an Sauerstoff im wesentlichen horizontal darübergeführt wurde. Hierbei handelt es sich jedoch nicht um eine Schnellveraschungsmethode, weil die in einem Mikrowellenofen erzeugte niedrige Temperatur hierfür gar nicht geeignet gewesen wäre, und anderseits lässt sich die Erhitzung mit Hilfe von Mikrowellen nicht ohne weiteres von tierischem auf pflanzliches Material übertragen.

Erfindungsgemäss wird nun ein Probenbehälter verwendet, der dadurch gekennzeichnet ist, dass der an seiner Oberseite eine Deckfläche besitzt, dass er zwei Oeffnungen für den im wesentlichen quer zu seiner Höhenabmessung verlaufenden Durchstrom eines Sauerstoff führenden Gases aufweist und dass wenigstens eine der Oeffnungen im wesentlichen dem lichten Querschnitt einer Stirnseite entspricht. Diese Merkmale in Kombination mit einer Temperatur oberhalb 750°C und der Verwendung eines entsprechend hitzebeständigen Materials für den Probenbehälter bewirken, dass nicht nur die Veraschung rasch vor sich geht, sondern dass der Probenbehälter auch leicht und rasch manipuliert werden kann, d.h. dass durch die wenigstens eine relativ grosse Oeffnung der Zutritt zum Inneren des Probenbehälters erleichtert ist und zusätzlich die Notwendigkeit eigener Gasanschlüsse an den Probenbehälter (die unter der Hitze besonders anfällig wären) entfallen kann.

Sobald man aber für eine Schnellveraschungsmethode auf die Verwendung besonderer Anschlüsse an den Probenbehälter zur dosierten Zufuhr von Sauerstoffgas verzichten will, ist mit einem herkömmlichen Veraschungsofen nur schlecht ein einigermassen reproduzierbares Ergebnis zu erhalten. Daher geht die Erfindung von einem Ofen mit einer eine Temperatur oberhalb 750°C erzeugenden Heizeinrichtung und einer durch eine Türe verschliessbaren Ofenkammer aus, und besteht im wesentlichen darin, dass die Ofenkammer zwei Oeffnungen für die Zu- und Abfuhr eines Sauerstoff enthaltenden Gases besitzt, das vorzugsweise die Ofenkammer einen Querschnitt aufweist, der im wesentlichen einer Flächenabmessung einer zumindest einen Probenbehälter abstützenden Trägerkonstruktion entspricht, und dass jeder Probenbehälter in der Trägerkonstruktion mit seinen beiden Oeffnungen im wesentlichen auf die Zu- und Abführöffnungen des Ofens ausgerichtet ist. In einer einfachen Ausführung könnte daher die Trägerkonstruktion einfach in einer Vertiefung der Auskleidung der Ofenkammer bestehen, in die wenigstens ein Probenbehälter eingelegt wird, wobei seine beiden Oeffnungen im wesentlichen auf die Zu- und Abführöffnungen des Ofens ausgerichtet sind. Durch diese Trägerkonstruktion wird erreicht, dass der Probenbehälter stets unter gleichen Bedingungen innerhalb der Ofenkammer auf die durch den Ofen ziehende Gasströmung ausgerichtet ist. Wenn dabei in der bevorzugten Weise die Ofenkammer einen Querschnitt aufweist, der im wesentlichen einer Flächenabmessung der Trägerkonstruktion entspricht, so ist damit gesichert, dass das den Sauerstoff enthaltende Gas im wesentlichen durch jeden Probenbehälter strömen muss. Hierbei ist es zwar vorteilhaft, jedoch nicht zwangsläufig erforderlich, dass diese Flächenabmessung tatsächlich von einer Baufläche der Trägerkonstruktion ausgefüllt ist, vielmehr handelt es sich um jene Flächenabmessung, die den Umrissen einer Stirn- oder Seitenansicht entspricht.

Dabei kann die Trägerkonstruktion zur Erleichterung der Manipulation durchaus auch aus der Ofenkammer herausnehmbar sein, beispielsweise nach Art von Schubladenführungen an Schienen herausführbar sein, bevorzugt ist sie jedoch als gesonderter Teil ausgebildet.

Während man für die bisher verwendeten Probenschälchen löffel- oder schalenartige Dosiereinrichtungen verwendete, wie dies beispielsweise in der DE-PS 711 639 beschrieben ist, ist dies bei einem erfindungsgemässen Probenbehälter insbesondere dann nicht günstig, wenn dieser im wesentlichen prismatisch oder in der bevorzugten Weise zylindrisch ausgebildet ist, weil dann der Inhalt der Dosiereinrichtung nur mit Mühe in den Probenbehälter eingebracht werden könnte. Darum ist eine erfindungsgemässe Dosiereinrichtung für einen solchen, im wesentlichen prismatischen Probenbehälter annähernd stabförmig mit einem der kleinsten lichten Querschnittsabmessung des im wesentlichen prismatischen Probenbehälter entsprechenden Querschnitt ausgebildet und weist eine die Probe aufnehmende Vertiefung auf, wobei vorzugsweise an der Vertiefung wenigstens eine Abstreifkante vorgesehen ist. Dadurch, dass die Dosiereinrichtung im wesentlichen stabförmig ausgebildet ist, kann sie leicht in den im wesentlichen prismatischen Probenbehälter eingeführt und die Materialprobe an die richtige Stelle im Inneren des Probenbehälters abgelegt werden. Die richtige Stelle befindet sich etwa in der Mitte bzw. sogar etwas gegen diejenige Oeffnung zu versetzt, von wo aus das den Sauerstoff enthaltende Gas zuströmt. Dadurch ist gesichert, dass auch bei Mitnahme von Aschenteilen durch das Gas diese Aschenteile unmittelbar hinter der Probe wieder abgelegt werden, wobei die prismatische Form genügend Platz ergibt, um eine solche Ablagerung zu gestatten. Dabei ist durch die Stabform der Dosiereinrichtung überdies gesichert, dass die Probe beim Herausziehen der Dosiereinrichtung nicht verformt wird, weil die stabförmige Dosiereinrichtung in dem Probenbehälter aufgrund der angegebenen Abmessungsverhältnisse wie in einem Lager geführt ist. Vorzugsweise ist die Dosiereinrichtung mit einer Abstreifkante versehen, einerseits um beim Einfüllen die vorbestimmte Menge zu garantieren, anderseits um beim Herausziehen aus dem Probenbehälter für eine vorbestimmte Form der Probe zu sorgen und auch von dieser Seite her zu einer Vergleichmässigung der Veraschungsbedingungen beizutragen.

Gemäss den obigen Ausführungen geht das erfindungsgemässe Verfahren der Schnellveraschung organischer Proben, vorzugsweise von - z.B. zerkleinertem - pflanzlichen Material, insbesondere von Mehl von einem solchen mit einer Temperatur oberhalb 750°C aus und besteht im wesentlichen darin, dass bei dieser Temperatur die Verbrennungsgase am Aufsteigen gehindert werden und ein Sauerstoff enthaltendes Gas im wesentlichen horizontal mit einem Durchsatz von unterhalb 5 l/min und Probe über die Probe strömen gelassen wird. Es hat sich als vorteilhaft herausgestellt, wenn der Durchsatz des Gases, insbesondere stufenweise, vermindert wird und so in einem ersten Zeitraum höher ist als danach.

Weitere Einzelheiten ergeben sich anhand der nachfolgenden
Beschreibung von in der Zeichnung schematisch dargestellten
Ausführungsbeispielen.

Fig. 1          veranschaulicht sämtliche Vorrichtungen, die
                im Rahmen des gegenständlichen Schnellver-
                aschungssystems Verwendung finden.

Fig. 1A         zeigt eine Dosiereinrichtung in einem Probenbehälter in jener Stellung, in der die Probe im
                Probenbehälter abgelegt wird.

Fig. 2          zeigt schematisch das Innere der Ofenkammer
                während der Verbrennung.

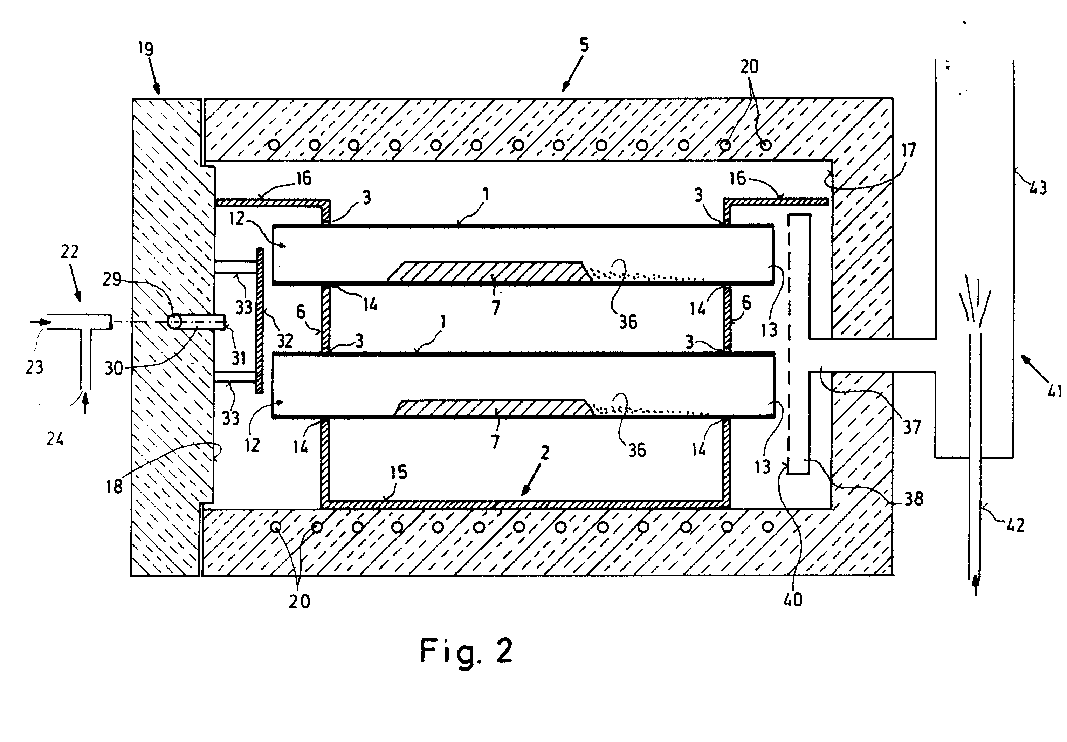

Mehrere röhrchenartige Probenbehälter 1 aus einem bei Temperaturen über 750°C, insbesondere um 900°C beständigem Material, wie Quarz, Siliziumkarbid od.dgl. werden auf einem im
wesentlichen U-förmigen Träger 2 in Löcher 3 derart eingeschoben,
dass die Probenbehälter 1 an zwei Enden so abgestützt sind,
wie dies aus Fig. 2 ersichtlich ist. Im dargestellten Ausführungsbeispiel weist der Träger 2 vier Oeffnungen 3 auf, deren
Innenflächen als Tragflächen für die Probenbehälter 1 dienen.
Diese Form des Trägers 2 ist aus mehreren Gründen besonders
vorteilhaft, obwohl es an sich auch möglich wäre, auf einer
Grundplatte Stäbe zu montieren, die an ihren oberen Enden
etwa halbmondförmige Tragflächen aufweisen. Es versteht sich
jedoch, dass die dargestellte U-Form einerseits leichter herstellbar ist, anderseits gegenüber der später noch besprochenen, den Ofen 4 bzw. dessen Ofenkammer 5 durchziehenden Strömung mit der Fläche des vorderen Schenkels 6 derart einen
Widerstand entgegensetzt, dass die Strömung zwangsläufig
ausnahmslos die Probehälter 1 am Träger 2 durchströmen muss.
Ausserdem ist diese Form auch bei hohen Temperaturen bzw. grossen Temperaturunterschieden äusserst stabil.

Zu Beginn einer Schnellveraschung muss eine genau dosierte
Menge des Probenmateriales 7 (Fig. 2) in der ersichtlichen Weise in die Probenbehälter eingebracht werden. Hierzu ist vorzugsweise eine im wesentlichen stabförmige Dosiereinrichtung 8 vorgesehen, die an ihrem Vorderende einen Ausschnitt 9 mit einer Vertiefung 10 aufweist. In diese Vertiefung wird Probenmaterial, insbesondere Mehl, unter einem vorbestimmten Druck eingebracht und kann an der glatten seitlichen Abstreifkante 11 so abgestreift werden, dass eine genaue Menge des Mehles gesichert ist.

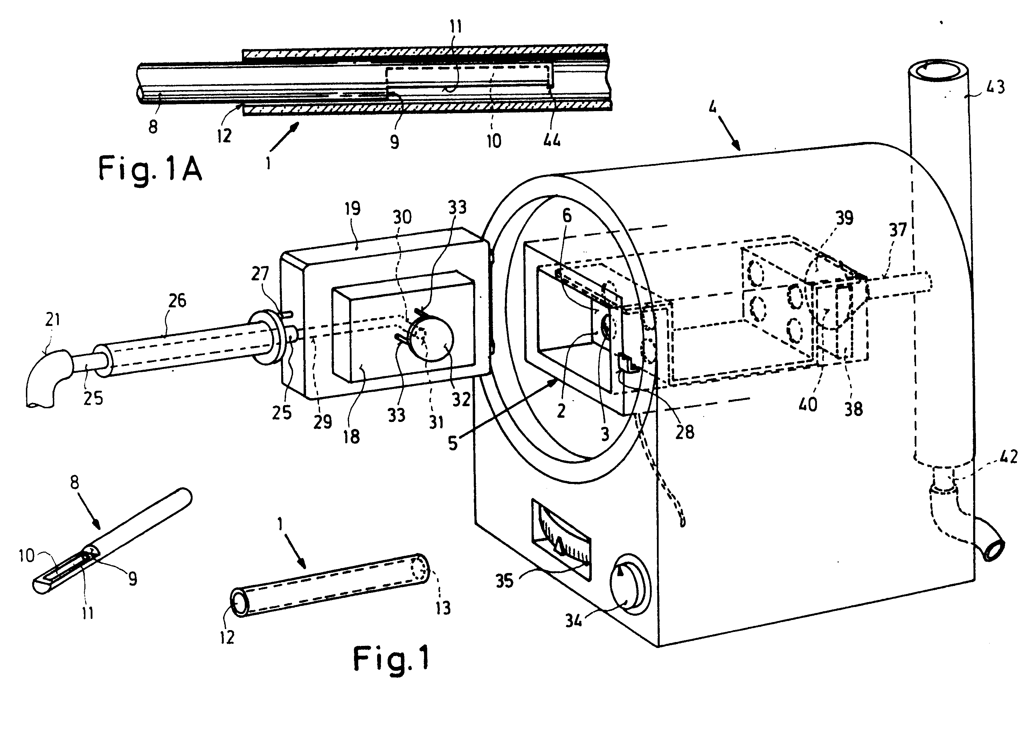

Sodann wird die Dosiereinrichtung 8 in einen Probenbehälter 1 eingeführt, wobei aus Fig. 1A ersichtlich ist, dass der Durchmesser der stabförmigen Dosiereinrichtung 8 im wesentlichen dem lichten Durchmesser des zylindrischen Probenbehälters 1 entspricht. Falls der Probenbehälter jedoch beispielsweise als Polygonprisma ausgeführt wird, so ist es selbstverständlich zweckmässig, den runden oder ebenfalls polygonen Stab der Dosiereinrichtung 8 entweder so auszuführen, dass er innerhalb des Probenbehälters gedreht werden kann oder (besser), dass er passend geformt wird und dann Probenbehälter samt Stab gedreht werden. Er gelangt dann in eine Lage, wie sie in Fig. 1A dargestellt ist, wobei das Probegut aus der Vertiefung 11 an der gewünschten Stelle des Probebehälters in diesen abgelegt wird.

Aus der oben beschriebenen Funktion der Dosiereinrichtung 8 sowie des Probenbehälters 1 ergibt sich, dass der Probenbehälter 1 an sich nur eine Oeffnung 12 aufzuweisen braucht, durch die die jeweilige Probe 7 (Fig. 2) einführbar bzw. die Asche leicht entfernbar ist. Die zweite Oeffnung 13 könnte an sich auch verschmälert, gewünschtenfalls aber auch verbreitert sein (etwa um am anderen Ende die Gasströmung zu verlangsamen). Ferner muss die Oeffnung 13 nicht zwangsläufig der Oeffnung 12 gegenüber liegen, vielmehr kann das Ende des Probehälters mit der Oeffnung 13 gegebenenfalls auch gekrümmt sein bzw. ist es möglich, die Auslassöffnung 13 in beliebiger Weise anzuordnen.

Sobald eine Anzahl von Probenbehältern 1 mit dem Probegut
versehen sind, werden sie in die Oeffnungen 3 des Trägers 2
eingeschoben und ruhen dort auf den Tragflächen 14 auf. Die
Anzahl der Probebehälter 1 hängt von der Art der gewünschten
Probe und der Aufnahmekapazität des Trägers 2 auf, der anstelle der vier gezeigten Oeffnungen 3 auch mehrere, z.B. zehn
oder eine geringere Anzahl aufweisen kann. Zur Erleichterung
des Einsetzens der Probebehälter 1 in den Träger 2 ist dieser
zweckmässig aus dem Ofen 4 herausnehmbar, obwohl es im Prinzip auch möglich wäre, die Tragflächen 14 in der Ofenkammer 5
starr anzuordnen.

Wie besonders aus Fig. 2 ersichtlich ist, besitzt der im wesentlichen U-förmige Träger 2 einen seine beiden Schenkel 6
verbindenden Bügel 15, der als Standfläche dient. Die freien
Enden der Schenkel 6 bilden vorzugsweise nach aussen abstehende Abwinkelungen 16, die einerseits die Enden der Probebehälter 1 schützend überdachen, anderseits zweckmässig so lange
ausgebildet sind, dass sie Anschläge einerseits gegen die
hintere Wand 17 der Ofenkammer 5 und anderseits gegen die
Innenfläche 18 der Ofentüre 19 bilden. Auf diese Weise wird
eine stets gleichbleibende Lage der Probebehälter 1 gesichert
und damit für eine Vergleichmässigung der Veraschungsbedingungen weiterhin gesorgt. Unter diesen Umständen zeigt sich
anhand der Fig. 2 deutlich, dass es nicht unbedingt erforderlich ist, die Vorderfläche des der Türe 19 zugekehrten
Schenkels 6 über die volle Höhe der Ofenkammer 5 reichen zu
lassen, da ja die Abwinkelung 16 mit verhindert, dass einströmendes Sauerstoffgas an den Probehältern 1 vorbeistreicht und
so ein Teil der dosiert zugeführten Sauerstoffmenge in unkontrollierter Weise verloren geht. Es ist also lediglich zweckmässig, wenn die Stirnwandkonturen des Trägers 2 im wesentlichen der Querschnittsfläche der Ofenkammer 5 entsprechen,
d.h. dem wesentlichen Teil dieser Fläche. Obwohl Abstandhalter auch auf andere Weise erzielt werden könnten, ist diese Ausführung besonders vorteilhaft, weil einerseits die Abwinkelungen 16 leicht herstellbar sind und zur Erzielung eines Trägers 2 im Prinzip ein Blech lediglich gestanzt und gebogen werden muss, anderseits deshalb, weil diese Abwinkelungen 16 hier auch als Gasabschirmung dienen, die in dieser Form günstiger ist, als wenn der Schenkel 6 über die volle Höhe der Ofenkammer 5 verliefe, woraus sich Toleranzprobleme ergeben könnten. Wenn auch Fig. 1 deutlich zeigt, dass der U-förmige Träger 2 hinsichtlich seiner Breitenabmessungen der vollen Breite der Ofenkammer 5 im wesentlichen entspricht, so ist doch auch hier eine ähnliche Abwinkelung an der Seite möglich, die dann in einer etwa lotrechten Ebene verlaufen würde, um so eine Abschirmung gegen Gasverlust zu erzielen und dennoch den Träger 2 schmäler ausbilden zu können. Andernseits kann eine Verschmälerung des Trägers 2 leicht dazu führen, dass er in die Ofenkammer 5 etwas schief eingesetzt wird, wodurch sich die Strömungsbedingungen verändern würden. Gerade deshalb ist die dargestellte einfache Form besonders günstig.

Allerdings könnte eine Schrägstellung des Trägers 2 auch bei schmälerer Ausführung dadurch verhindert werden, dass entsprechende Führungsschienen in der Ofenkammer vorgesehen sind, die gleichzeitig das Herausschieben des Trägers 2 gestatten.

Die Ofenkammer 5 besitzt eine Heizeinrichtung, die beispielsweise von in die Wandung eingebetteten elektrischen Heizspiralen 20 gebildet sein kann. Die Art der Heizeinrichtung ist an sich unerheblich, doch ist wesentlich, dass sie eine Erwärmung auf die gewünschte Temperatur oberhalb 750°C und vorzugsweise unterhalb 1100°C, insbesondere auf 900°C sichert. Innerhalb der Ofenkammer sollte die Temperaturverteilung wenigstens so gleichmässig sein, dass alle in die Kammer 5 eingebrachten Probenbehälter 1 im Bereiche der Proben 7 jeweils die gleiche Temperatur besitzen. Es ist dabei ebenso
unerheblich, ob der Träger 2 in der dargestellten Weise der
Länge nach in die Ofenkammer 5 eingebracht wird oder ob der
Ofen 4 so konstruiert ist, dass der Träger 2 quer zu stehen
hat.

In jedem Falle ist es aber vorteilhaft, wenn die Probenbehälter 1 so angeordnet sind, dass ihre Eingangsöffnung 12 einer
Zufuhröffnung für ein Sauerstoff tragendes Gas zugewandt ist.
Falls die Probebehälter 1 in Längsrichtung in der gezeigten
Art in die Ofenkammer 5 geschoben werden, ist es zweckmässig,
wenn die Zufuhröffnung an der Türe 19 angebracht ist, obwohl
die Konstruktion auch so gewählt sein kann, dass von einer
Seitenwand her ein Rohr gegen die Mitte ragt. Dieses Rohr
müsste aber dann wegschwenkbar sein, was Dichtungsprobleme
ergibt. Falls hingegen die Probebehälter 1 quer in die Ofenkammer 5 eingeschoben werden, so muss selbstverständlich die
Zu- und Abfuhröffnung für das Gas im allgemeinen an den Seitenwänden vorgesehen sein.

Als Sauerstoff tragendes Gas kommt vor allem Luft in Frage,
die beispielsweise über eine (nicht dargestellte) Druckpumpe
und einen Schlauch 21 (Fig. 1) zugeführt werden kann. Es kann
jedoch auch an den Schlauch 21 ein Düsensystem 22 (Fig. 2)
angeschlossen sein, durch dessen eine Oeffnung 23 Luft strömt
und aus einer Seitenöffnung 24 reinen Sauerstoff mitreisst.

Der Schlauch 21 ist an ein zum Griff der Türe 19 gehörendes
Rohr 25 angeschlossen. Das Rohr 25 ist zweckmässig verhältnismässig lang ausgebildet, um bis zur Anschlussstelle mit
dem Schlauch 21 genügend Wärme abzugeben. Um andererseits
die manuelle Betätigung der Türe 19 zu ermöglichen, ist zweckmässig eine Hülse 26 aus isolierendem Material am Rohr 25

- 11 -

vorgesehen. Gewünschtenfalls kann zwischen isolierender Hülse 26 und Rohr 25 ein entsprechender Luftabstand vorhanden sein, um eine Wärmeabstrahlung zu ermöglichen.

Vorteilhaft ist die Isolierhülse 26 mit dem Verschluss für die Türe 19 vereinigt. Zu diesem Zwecke ist die Hülse 26 am Rohr 25 drehbar, jedoch axial unverschieblich gelagert. An ihrem der Türe 19 zugewandten Stirnende besitzt sie einen Drehriegelzapfen 27, der zum Verschliessen der Türe in eine Halterung 28 eingreift. Gegebenenfalls kann die Isolierhülse 26 auch mit einem (nicht dargestellten) Ventil für das Rohr 25 versehen sein, das beispielsweise zwischen Hülse 26 und Schlauch 21 angeordnet ist und von einer an der hinteren Stirnfläche der Hülse 26 angeordneten Nocke derart betätigt wird, dass das Ventil bei geöffneter Türe bzw. bei in Offenstellung befindlichen Riegel 27 geschlossen ist und erst beim Verriegeln der Türe 19 das Rohr 25 frei gibt.

Das Rohr 25 ist fluchtend mit einer Bohrung 29 in der Türe 19 verbunden. Etwa in der Mitte der Türe 19 besitzt die Bohrung 29 eine abgewinkelte Abzweigung 30 (vgl. Fig. 2), die im Bereiche der Innenwand 18 der Türe 19 mündet (Mündung 31).

Zur besseren Verteilung der einströmenden Luft mag es zweckmässig sein, im Bereiche der die Zufuhröffnung darstellenden Mündung 31 eine Diffusoreinrichtung vorzusehen. Dies kann in der Weise erfolgen, wie dies von Klimaanlagen her bekannt ist, es kann um die Mündung 31 diffundierbares Material, wie Quarzwolle, Sintermaterial od.dgl. angeordnet werden und/oder es kann einfach eine Diffusorplatte 32 an einen vorbestimmten und gegebenenfalls einstellbaren Abstand sichernden Füssen 33 befestigt sein, die aus dem Material der Türe 19 ragen.

Sobald nun die Ofenkammer 5 durch Einschalten der Heizung 20 über einen Schaltknopf 34 auf die erforderliche Temperatur gebracht ist (was an einem Thermometer 35 abgelesen werden kann)
und mit Probenbehältern beschickt ist, wird Luft, ein Luft-Sauerstoffgemisch oder reiner Sauerstoff durch die Zufuhröffnung 31
in die Kammer geblasen. Wie schon erwähnt, erfolgt die Zufuhr
in vorsichtig dosierter Weise.   Die Durchsatzgeschwindigkeit muss deshalb so gewählt werden, dass die durch die
Probenbehälter 1 fliessende Strömung die Asche 36 im Anschluss
an die Materialprobe 7 wieder absetzt. Dies wird dadurch erleichtert, dass die Probe 7 eine Verengung des Durchflussquerschnittes bewirkt und die anschliessende Erweiterung das
Absetzen etwaiger mitgerissener Ascheteilchen begünstigt. Gewünschtenfalls kann dieser Effekt noch dadurch gefördert werden, dass die Probenbehälter 1 in diesem Bereiche selbst eine
Erweiterung aufweisen. In der Praxis hat es sich gezeigt,
dass ein Durchsatz unterhalb von 10 l/min, insbesondere zwischen 6 und 8 l/min, am günstigsten sind.

Gegenüber der Auslassöffnung 13 der Probenbehälter 1 ist an
der Hinterwand 17 der Ofenkammer 5 eine Abfuhröffnung 37 vorgesehen, die sich im Inneren der Ofenkammer 5 und gegenüber
den Auslassöffnungen 13 der Probebehälter 1 zu einem Kasten 38
erweitert. Diese Erweiterung ist vorzugsweise trichterförmig
ausgebildet, wie dies in Fig. 1 anhand eines angedeuteten
Trichters 39 ersichtlich ist. Der Kasten 38 ist durch eine
Lochplatte 40, ein Gitter oder Sieb abgedeckt. Dies ist deshalb von Vorteil, weil zweckmässig an die Abfuhröffnung 37
eine Saugpumpe 41 angeschlossen ist und so deren Saugwirkung
besser verteilt wird. Dabei sorgt die hintere Abwinkelung 16
des Trägers 2 dafür, dass der Träger 2 den richtigen Abstand
gegenüber der Hinterwand 17 besitzt, um seinerseits einen entsprechenden Abstand der Auslassöffnungen 13 von der Gitter-

0145863

- 13 -

platte 40 zu sichern.

Die Saugpumpe 41 kann an sich beliebiger Art sein, doch hat sich der aus Fig. 2 ersichtliche Typ als zweckmässig erwiesen, bei dem an ein Injektorrohr 42 eine Druckluftquelle angeschlossen ist. Das Injektorrohr 42 mündet im Inneren eines Kaminrohres 43 nahe der Abfuhröffnung 37 und entfaltet so seine Saugwirkung. Es versteht sich aber, dass gegebenenfalls entweder auf eine Druckmittelquelle in Verbindung mit der Zufuhröffnung 31 oder auf eine Saugpumpe 41 verzichtet werden kann. Obwohl die Zufuhr unter Druck und die Abfuhr durch Saugwirkung bevorzugt ist, erscheint es günstiger, bei Verzicht auf eine der Pumpenanordnungen wenigstens die Saugpumpe 41 beizubehalten.

In der Praxis hat sich gezeigt, dass mit dem erfindungsgemässen Schnellveraschungssystem repräsentative Werte innerhalb einer Viertelstunde oder darunter erhältlich sind. Es ist somit mit einem derartigen System eine genauere und raschere Kontrolle als bisher möglich.

Im Rahmen der Erfindung sind zahlreiche verschiedene Ausführungen denkbar; so sei darauf hingewiesen, dass gemäss Fig. 1A der Dosierstab 8 an seiner vorderen Stirnseite zusätzlich zur Abstreifkante 11 eine weitere Abstreifkante 44 besitzt, die beim Herausziehen der Dosiereinrichtung 8 in der aus Fig. 1A ersichtlichen Lage das Glattstreichen der Oberfläche der Proben 7 (Fig. 2) bewirkt, so dass die Proben 7 in den Probenbehältern 1 letztlich eine einheitliche Form besitzen und so die Probebedingungen auch in dieser Hinsicht einheitlich sind. Es versteht sich jedoch, dass eine solche Abstreifkante 44 zwar vorteilhaft, jedoch nicht unbedingt erforderlich ist.

Es mag auch ein Teil der in der dargestellten Ausführung von der Abwinkelung 16 gebildeten Ueberdachung an der Innenwand 18 angeordnet sein und mit einer entsprechend kürzeren Abwinkelung 16 oder mit einem Schenkel 6 zur Wahrung des richtigen Abstandes zusammenwirken. Dies wird insbesondere dann der Fall sein, wenn die Diffusoreinrichtung in einem Gehäuse mit entsprechend vorragenden, zum Abstandhalten dienenden Wänden angeordnet ist, etwa im Falle der Hintereinanderschaltung mehrerer Diffusorplatten, einer (z.B. zyklonartiges oder einen schraubenförmigen Einlass aufweisenden) Verwirbelungseinrichtung oder einer, z.B. nach Art eines Segner'schen Rades, drehbaren Luftverteilungseinrichtung. Eine Diffusoreinrichtung kann aber entfallen, wenn wenigstens eine Probe eine gesonderte Zu- und/oder Abfuhröffnung zugeordnet ist, wobei eine Dosierung für jede Probe besonders leicht ist.

Wie oben bereits erwähnt, ist es meist nützlich, zu Beginn der Veraschung die Sauerstoffzufuhr zu verstärken und erst später zu drosseln. An sich kann das Drosseln kontinuierlich erfolgen, doch wird es günstiger sein, erst während eines vorbestimmten ersten Zeitraumes eine gleichbleibende oder nur leicht abnehmende Menge an Gas einzublasen und anschliessend die Sauerstoffzufuhr stärker zu vermindern. Dies könnte an sich auch in mehr als zwei Stufen erfolgen, doch wird dadurch im allgemeinen kein zusätzlicher Vorteil erzielt. Am vorteilhaftesten ist es dabei, wenn der erste Zeitraum bzw. die erste Stufe kürzer gehalten wird, als der Rest der Veraschungszeit, so dass er unter der Hälfte der Gesamtzeit liegt. Ein Zeitraum von etwa einem Viertel (und darunter) der Gesamtzeit hat sich als ausreichend erwiesen, wobei sich in der Praxis weniger als 4 min., z.B. 2 min, ergeben.

Bis zu einem gewissen Grade hängt die Dauer der Stufen auch vom Gasdurchsatz ab, der im ersten Zeitraum (allenfalls im

- 15 -

Durchschnitt) zwischen 2 und 4 l/min und Probe betragen kann,
danach zwischen 0,5 und 2 l/min und Probe. Als Faustregel kann
die Einstellung im Anschluss an den ersten Zeitraum etwa auf
die erste Hälfte des Gasdurchsatzes während dieses ersten Zeitraumes zurückgestellt werden. Hierfür kann der Ofen vorteilhaft mit einer zeitabhängig gesteuerten Programmeinrichtung
versehen sein.

PT/Rv/hb
17.08.84

PATENTANSPRUECHE

1. Probenbehälter für Veraschungsproben, vorzugsweise von z.B. zerkleinertem pflanzlichen Material, insbesondere von Mehl, bestehend aus einem gegen Temperaturen oberhalb 750°C hitzebeständigen Material, und in einer in Seitenansicht mehrfach breiteren als hohen Form, dadurch gekennzeichnet, dass der Probenbehälter (1) an seiner Oberseite eine Deckfläche besitzt, dass er zwei Oeffnungen (12, 13) für den im wesentlichen quer zu seiner Höhenabmessung verlaufenden Durchstrom eines Sauerstoff führenden Gases aufweist, und dass wenigstens eine der Oeffnungen (12) im wesentlichen dem lichten Querschnitt einer Stirnseite entspricht.

2. Behälter nach Anspruch 1, dadurch gekennzeichnet, dass er (1) im wesentlichen prismatisch, vorzugsweise zylindrisch ausgebildet ist, und dass er insbesondere rohrförmig mit je einer Oeffnung (12, 13) an seinen beiden Stirnenden ausgebildet ist.

3. Veraschungsofen für Probenbehälter nach Anspruch 1 oder 2, mit einer eine Temperatur oberhalb 750°C erzeugenden Heizeinrichtung und einer durch eine Türe verschliessbaren Ofenkammer, dadurch gekennzeichnet, dass die Ofenkammer (5) zwei Oeffnungen (31, 37) für die Zu- und Abfuhr eines Sauerstoff enthaltenden Gases besitzt, dass vorzugsweise die Ofenkammer (5) einen Querschnitt aufweist, der im wesentlichen einer Flächenabmessung einer zumindest einen Probenbehälter (1) abstützenden Trägerkonstruktion (2) entspricht, und dass jeder Probenbehälter (1) in der Trägerkonstruktion (2) mit seinen beiden Oeffnungen (12, 13) im wesentlichen auf die Zu- und Abführöffnungen (31, 37) des Ofens (4) ausgerichtet ist, dass bevorzugt bei mehreren einer Oeffnung zugeordneten Proben wenigstens zwischen einer Oeffnung (31) und der Probe (7) eine Diffusoreinrichtung (32) für das Gas vorgesehen ist, und dass insbesondere, vorzugsweise vor der Zufuhröffnung (31), in der Ofenkammer (5) eine den Oeffnungsquerschnitt abdeckende Diffusorplatte (32) vorgesehen ist.

4. Ofen nach Anspruch 3, dadurch gekennzeichnet, dass wenigstens einer Probe (7) zumindest eine gesonderte Zu- bzw. Abfuhröffnung zugeordnet ist, durch die Gas nur dieser Probe zuführbar bzw. von ihr abführbar ist.

5. Ofen nach Anspruch 3 oder 4, dadurch gekennzeichnet, dass eine seiner Oeffnungen (31) an der Türe (19) angeordnet ist, und dass vorzugsweise eine Zufuhrleitung (25) für das Gas durch die Ofenverschlusshandhabe (26) verläuft, welcher Verschluss zweckmässig als Drehverschluss (27, 28) ausgebildet ist, wobei gegebenenfalls als der Ofenverschluss mit einem Ventil zum Sperren der Gasabfuhr bei geöffnetem Ofenverschluss verbunden ist.

6. Ofen nach Anspruch 3, 4 oder 5, dadurch gekennzeichnet, dass die Trägerkonstruktion (2) aus der Ofenkammer (5) herausnehmbar, insbesondere als gesonderter Teil ausgebildet ist, und/oder dass die Trägerkonstruktion (2) im wesentlichen U-förmig ausgebildet ist, und dass jeder Schenkel (6) dieser U-förmigen Konstruktion (2) zumindest eine Tragfläche (14) für einen Probenbehälter (1) aufweist,

dass zweckmässig am freien Ende wenigstens eines Schenkels (6) der Trägerkonstruktion (2) eine das über die Tragfläche (14) vorragende Ende des Probenbehälters (1) Ueberdachung (16) und/oder ein Abstandhalter (16) von der Ofenwand (18) bzw. einem damit verbundenen Teil vorgesehen ist, die bevorzugt als Abwinkelung (16) des Schenkels (6) ausgebildet ist,

dass gegebenenfalls die Tragfläche (14) an einer den Probenbehälter (1) aufnehmenden Oeffnung (3) im Schenkel (6) ausgebildet ist,

und dass beispielsweise an jedem Schenkel (6) mehrere Tragflächen (14) für eine Anzahl von Probenbehältern (1) vorgesehen sind.

7. Dosiereinrichtung für einen Probenbehälter nach Anspruch 2, dadurch gekennzeichnet, dass sie (8) annähernd stabförmig mit einem der kleinsten lichten Querschnittsabmessung des im wesentlichen prismatischen Probenbehälters (1) entsprechenden Querschnitt ausgebildet ist, dass sie eine die Probe aufnehmende Vertiefung (10) aufweist, und dass vorzugsweise an der Vertiefung wenigstens eine Abstreifkante (11, 44) vorgesehen ist.

8. Einrichtung nach Anspruch 7, dadurch gekennzeichnet, dass sie (8) zylindrisch ausgebildet ist.

9. Verfahren zur Schnellveraschung organischer Proben, vorzugsweise von - z.B. zerkleinerten - pflanzlichen Material, insbesondere von Mehl, bei einer Temperatur oberhalb 750°C, dadurch gekennzeichnet, dass bei dieser Temperatur die Verbrennungsgase am Aufsteigen gehindert werden und ein Sauerstoff enthaltendes Gas im wesentlichen horizontal mit einem Durchsatz unterhalb 5 1/min und Probe über die Probe (7) strömen gelassen wird.

10. Verfahren nach Anspruch 9, dadurch gekennzeichnet, dass der Durchsatz des Gases, insbesondere stufenweise vermindert wird und so in einem ersten Zeitraum höher ist als danach,

dass vorzugsweise - bei stufenweiser Veränderung - der der höheren Stufen entsprechende erste Zeitraum weniger als die Hälfte der gesamten Veraschungsdauer, gegebenenfalls unter 4 min., z. B. 2 min., beträgt,

und dass zweckmässig der Gasdurchsatz im ersten Zeitraum zwischen 2 und 4 l/min und Probe, und danach etwa die Hälfte bzw. 0,5 bis 2 l/min und Probe beträgt.

PT/Rv/hb
29.08.84

Fig. 1A

Fig. 1

1/2

0145863

Fig. 2

0145863